# EUROPEAN PATENT APPLICATION

(11) **EP 1 749 588 A2**
(43) Date of publication of application: **07.02.2007**
(21) Application number: 06016320.1
(22) Date of filing: 04.08.2006
(51) Int. Cl.: B06B 1/10, A61B 8/06

(54) **Diagnostic apparatus**

(30) Priority: 04.08.2005 GB 0516067
(71) Applicant: UGCS(UNIVERSITY OF GLAMORGAN COMMERCIAL SERVICES LIMITED), Mid Glamorgan CF37 1DL (GB)
(72) Inventor: McCarthy, Peter William, Pontypridd Mid Glamorgan, CF37 1DL (GB); Heusch, Andrew Ian, Pontypridd Mid Glamorgan, CF37 1DL (GB)
(74) Representative: Haslam, Simon David

(57) **Abstract**

An apparatus (500) for the diagnosis of neurovascular disease of at least part of an upper limb of a subject, the apparatus comprising:
(i) a device (1) for inducing vibration in at least part of an upper limb of a subject, said device comprising a handle (2a, 2b) for the subject to grasp, wherein the handle is for vibrating at least part of an upper limb of the subject and
(ii) a sensor (400) for measuring a parameter from which a measure of the bloodflow in at least part of the upper limb may be derived. A method of measuring a parameter from which a measure of bloodflow of a subject may be derived is also provided.

## Description

The present invention relates to an apparatus for the diagnosis of diseases potentially caused by vibration to the hand and/or arm, in particular, but not exclusively, hand arm vibration syndrome (HAVS), including vibration white finger (VWF).

Use of vibrating tools and machinery is implicated in the aetiology of damage to the skeletal and neurovascular systems. People using vibrating machinery (such as drills, sanders, bicycles and even motorcycles) over prolonged periods can experience blanching of one or more digits in response to continued use of the respective equipment. Other neurological symptoms may be experienced, such as paraesthesia, pain, loss of function of the digit, and in extreme circumstances, necrosis leading to amputation or possibly death.

A number of tests are currently used to attempt to diagnose vibration-induced illnesses with a neuro-vascular involvement, tests such as cold provocation tests, grip strength test, dexterity test, thermal aesthesiometry, vibrotactile threshold test, arterial circulation speed test, finger vibration test and blanching test.

A typical cold provocation test is to immerse the hand (possibly in a plastics bag) into cold water for a predetermined period of time (usually between about 1 and 5 minutes) and measure the period of time that the hand takes to return to its pre-immersion temperature, the temperature being measured or monitored by instrument (such as thermography) or visually. If the hand takes much longer time than usual to return to the pre-immersion temperature, then this is indicative of damage to the neurological and/or vascular systems. An improved test using gas as a cooling agent rather than water is described in GB2203835. A typical grip strength test measures the strength of the grip of the subject, a weakened grip indicating that the hand and/or forearm may be damaged. A typical dexterity test involves an assessment of the manual dexterity of the subject using a pegboard, a poor performance indicating possible damage to the hands of the subject. Thermal aesthesiometry measures the subject's perception of hot and cold, readings typically being taken from the index finger and the little finger. A decreased sensitivity to one or both of hot and cold temperatures indicates damage to the nervous system. The vibrotactile test involves measuring the sensitivity of the finger of the subject to vibratory stimuli (such as the test described in US5230345); various frequencies may be used. Many of these tests, especially the ones that are reliant upon the patient reporting a result, are very subjective and dependent on the reporting bias of the patient. The abstract of SU1436983 discloses a device and method for the diagnosis of vibration-induced disease wherein a finger is vibrated and the electroluminescence of the finger measured. In order to investigate the disease status of all the digits, then each digit would have to be individually tested. This is time consuming and possibly arduous for the subject or patient.

The present invention seeks to reduce some of the problems associated with the prior art and provide an alternative diagnostic apparatus for certain neurovascular conditions.

There is provided in accordance with the present invention an apparatus for the diagnosis of neurovascular disease of at least part of an upper limb of a subject, the apparatus comprising:
(i) a device for inducing vibration in at least part of an upper limb of a subject, said device comprising a handle for the subject to grasp, wherein the handle is for vibrating at least part of an upper limb of the subject, and
(ii)a sensor for measuring a parameter from which a measure of the bloodflow in at least part of the upper limb may be derived.

The bloodflow may be indirectly or directly derivable from, if desired, from the parameter measured by the sensor, although it is not an essential element of the invention for a measure of the bloodflow to actually be derived.

The sensor for measuring the parameter from which a measure of the bloodflow may be derived may be in the form of a sensor for sensing bloodflow.

The apparatus may aid in the diagnosis in diseases and disorders such as HAVS and VWF.

The upper limb is understood to refer to the hand, forearm and upper arm.

Thus unlike any of the prior art mentioned herein the subject may be exposed to a stimulus of the type that may cause the disease or disorder.

The device for inducing vibration may comprise a handle for each hand of the subject. This allows the disease status of both hands of a subject to be investigated.

The or each handle may be provided with a force sensor for sensing the strength of the grip of the subject. This allows the strength of one or both of the hands of a subject to be monitored.

The apparatus may further comprise at least one indicator for indicating the strength of the grip of the subject, the at least one indicator being in communication with the force sensor. This informs the user of the strength of grip. In use, the indicator may indicate when the strength of the grip of the subject has reached a pre-determined value. This allows the subject to try to maintain a grip strength of the same, or greater than, the pre-determined value. The pre-determined value may be obtained by measuring the maximum grip strength of the subject and setting the pre-determined value to be a fixed percentage of the maximum grip strength. The fixed percentage may be, for example, from about 50% to 90%, preferably from about 60% to about 80%, and more preferably about 80%.

In use, if the strength of grip of the subject is below a pre-determined limit for longer than a pre-determined time, then the device for inducing vibration is deactivated. Testing may therefore be shortened for subjects with an unacceptably low ability of maintaining grip strength (which in itself may be indicative of the presence of vascular and/or neurovascular disease).

The indicator may comprise one or more of a visual display unit (such as a computer monitor or television screen), a light emitting diode, a light bulb and a sound-emitting device. The light emitting diode may be arranged to emit light when the strength of grip is greater than, or alternatively less than, the pre-determined level. A multicolour LED may be arranged to emit light of differing colours based on the grip strength of the subject in relation to the pre-determined level.

The apparatus may further comprise a controller, wherein the controller is in communication with one or more of the device for inducing vibration, the force sensor, the bloodflow sensor and the indicator. The controller may be in communication with the device for inducing vibration and the one force sensor, such that on the absence or presence of a pre-determined signal or series of signals from the said at least one force sensor, then the controller causes the device for inducing vibration to cease vibration. For example, the apparatus may be arranged, in use, to monitor whether the strength of the grip of the subject is below a pre-determined level for longer than a pre-determined time. If this is the case, then the controller may cause the device for inducing vibration to cease vibration. This may be achieved, for example, by the controller reducing the voltage to a motor that causes the vibrations. In the case where the device comprises two handles, each of which is provided with a force sensor, it is preferred that the controller is in communication with both of the force sensors.

The controller may be in communication with the force sensor and the indicator such that the controller provides signals to the indicator so that it may indicate the strength of grip of the subject. In the case where the device comprises two handles, each of which is provided with a force sensor, it is preferred that the controller provides signals to the indicator so that it may indicate the strength of grip in each hand of the subject.

The or each handle may be associated with a mechanism for vibrating the handle. The mechanism may comprise a cam and cam follower arrangement. This converts rotational movement of an axle into vibratory movement of said handle or handles. This is a convenient mechanism for generating vibration of the handle or handles. The or each handle may be associated with the cam follower, and the cam follower may be associated with a bias means that urges the cam follower into contact with the cam. This ensures that, in use, the cam follower maintains contact with the cam. Alternatively, there may be no bias means urging the cam follower into contact with the cam. In this case, the motion of the cam follower (and thus the handle or handles) may differ from when the bias means is present and may be less predictable.

The bias means may comprise a spring, for example a spring in compression or tension. Helical springs may conveniently be used.

As an alternative to the cam and cam follower, the mechanism for vibrating the handle may comprise an eccentrically mounted crank shaft, the rotation of which causes vibrational motion of the handle or handles. The handle or handles may be associated with a connecting arm that is connected to the crank shaft. Rotation of the crank shaft causes translational motion of the connecting arm, causing vibratory motion of the handle or handles. This provides a simple and effective mechanism for generating vibration of the handle or handles. The crank shaft may comprise a rotatable axle provided with a crank arm extending radially therefrom, the connecting arm being connected to the crank arm. The connecting arm may be movably connectable along the length of the crank arm. This allows the amplitude of vibration of the handle or handles to be varied.

As a further alternative, the mechanism for vibrating the or each handle may comprise an oscillatable mass associated with the handle or handles, the mass transfer of which during oscillation causes the or each handle to vibrate. The oscillatable mass may be located in proximity to, or even within, the or each handle.

The mechanism for vibrating the or each handle may comprise a shaker. The or each handle may be connected to or integral with a platform with which the shaker is associated. The platform may be in contact with the shaker. For example, the shaker may be placed on top of the platform. The movement of the shaker causes the platform and handles to vibrate. Said platform may be associated with one or more elastically-deformable members (such as one or more springs) which, in use, permit the platform to vibrate. For example, the platform may rest on one or more elastically-deformable members. The one or more elastically-deformable members effectively isolate the platform and shaker from any adjacent structure (for example, the table or plinth on which the device may be situated) so that the platform and shaker may be vibrated, without significant vibration of the adjacent structure.

The frequency of vibration may be from about 5Hz to about 1000Hz, preferably from about 5Hz to 500Hz and more preferably about 20Hz and/or about 200Hz.

The frequency of vibration may be variable. This may be achieved, for example, by altering the voltage applied to a motor that forms part of the mechanism for vibrating the handle or handles, thus varying the rotation speed of the motor and hence any axle connected thereto.

The amplitude of vibration of the handle or handles may be varied. If the mechanism for vibrating the or each handle or handles comprises a cam, then amplitude of vibration may be achieved by changing the size or shape of the cam.

The amplitude of vibration may be selected from about 2mm to 20mm and preferably from about 5mm to 10mm.

The device for inducing vibration may be arranged so as to produce vibrations substantially towards and away from the subject. Alternatively, the device may be arranged so as to produce vibrations in a substantially up-and-down motion.

The sensor for measuring a parameter from which a measure of the bloodflow in at least part of an upper limb may be derived may comprise a Doppler ultrasound sensor. The sensor for measuring a parameter from which a measure of the bloodflow in at least part of an upper limb may be derived may sense a parameter related to bloodflow, such as temperature or colour of the skin. A colorimeter may be used to sense the colour of the skin. Various sensors may be used to sense temperature, such as an infra-red camera or a thermometer or radiometer placed in contact with the skin of the subject or used at a measured distance from the skin without contact.

The invention further provides a device for inducing vibration in at least part of an upper limb of a subject suitable for use in an apparatus in accordance with the present invention. The device for inducing vibration may comprise those features described in relation to the apparatus in accordance with the present invention.

The invention further provides a method of measuring a parameter from which a measure of bloodflow of a subject may be derived, the subject having an upper limb with a hand, the method comprising:
(i) providing a device with a vibratable handle;
(ii) the subject grasping the handle;
(iii) causing the grasped handle to be vibrated; and
(iv) during or subsequent to vibration of the hand, measuring a parameter in part of the upper limb from which a measure of the bloodflow may be derived.

This provides an effective method of measuring a parameter from which a measure of bloodflow in part of the upper limb of a subject may be derived.

The parameter so measured may be for use in a method of diagnosis of a neurovascular disease in at least part of an upper limb of a subject.

The bloodflow may be indirectly or directly derivable from the measured parameter. Note that it is not a requirement of the method of the present invention for the measure of bloodflow to actually be derived.

Step (iv) may comprise measuring said parameter as a function of time.

Step (iv) may comprise making a first measurement of the parameter in a first part of the upper limb (preferably the hand) and making a second measurement of the parameter in a second (different) part of the upper limb (preferably a different part of the hand from the first part of the upper limb).

Step (iv) may comprise making a first set of measurements of the parameter in a first part of the upper limb and making a second set of measurements of the parameter in a second (different) part of the upper limb as a function of time. This is a particularly effective method.

The first and second parts of the upper limb may be distal and proximal regions of the same digit respectively, or may be a region of a digit and a region of the dorsum or palm respectively.

The method may further comprise the step of making a first measurement of said parameter in the first part of the upper limb and making a second measurement of said parameter in the second part of the upper limb before step (ii), the difference in the first and second measurements being a pre-stimulation difference.

Step (iv) preferably takes place subsequent to step (iii). It is more preferred that step (iv) commences immediately subsequent to step (iii).

The method may further comprise sensing the grasping force that the subject exerts on said handle during vibration. This allows grip strength to be sensed and measured.

The method may further comprise indicating the grasping force to the subject. This allows the subject to observe the grasping force. Indicating the grasping force to the subject may comprise indicating the magnitude of the grasping force relative to a predetermined level. This allows the subject to observe whether the grasping force is too low or too high.

The method may comprise the step of comparing the grasping force to a pre-determined value. For example, the grasping force may be compared to said pre-determined value over a period of time. In the event that the grasping force is lower than the pre-determined value for a period longer than a given pre-determined length of time, then the handle ceases to vibrate. This allows the method to be stopped in the event that the subject cannot maintain a sufficiently strong grip.

The frequency of vibration may be approximately constant throughout step (iii).

The frequency of vibration may be from about 5Hz to about 1000Hz, preferably from about 5Hz to 500Hz and more preferably about 20Hz and/or about 200Hz.

The amplitude of vibration may be from about 2mm to 20mm and preferably from about 5mm to 10mm.

The amplitude of vibration may remain substantially constant throughout step (iii). Alternatively, the amplitude of vibration may be varied throughout step (iii).

If step (iii) is performed using a first frequency of vibration, then the method may further comprise the step of repeating steps (ii) to (iv) after a pre-determined period of time, wherein a second frequency of vibration is used during repeated step (iii).

The second frequency is preferably different from the first frequency, and it is further preferred that the second frequency is lower than the first frequency.

The method of the present invention may use the apparatus in accordance with the present invention. Furthermore, the device provided in step (i) of the method of the present invention may have the features of the device for inducing vibrations in accordance with the present invention.

The results obtained from the method of measuring a parameter from which a measure of bloodflow may be derived may be used to indicate the presence or absence (and possibly severity) of neurovascular disease of the upper limb, such as HAVS. The use of the handle has proved to be particularly advantageous in the present case.

The invention further provides a method of diagnosing a neurovascular disease in at least part of an upper limb of a subject, said upper limb comprising a hand, the method comprising:
(i) providing a device with a vibratable handle;
(ii) said subject grasping said handle;
(iii) causing the grasped handle to be vibrated; and
(iv) during or subsequent to vibration of said hand, measuring a parameter in part of the upper limb from which a measure of the bloodflow may be derived, said measurement being indicative of the absence or presence of said neurovascular disease.

This method is particularly effective for diagnosing neurovascular diseases of the hand or arm, such as HAVS and VWF. The vibration of the handle mimics the type of vibration that could cause the disease or disorder to occur.

The measurement of said parameter may be suggestive of the absence, presence or severity of said neurovascular disease.

The bloodflow may be indirectly or directly derivable from the measured parameter. Note that it is not a requirement of the method of the present invention for the measure of bloodflow to actually be derived.

Step (iv) may comprise measuring said parameter as a function of time. In this case, the variation in time of said parameter may be suggestive of the absence, presence or severity of said neurovascular disease.

Step (iv) may comprise making a first measurement of the parameter in a first part of the upper limb (preferably the hand) and making a second measurement of the parameter in a second (different) part of the upper limb (preferably a different part of the hand from the first part of the upper limb). In this case, the method may further comprise comparing said first and second measurements, a difference between said first and second measurements may be suggestive of the absence, presence or severity of said neurovascular disease.

Step (iv) may comprise making a first set of measurements of the parameter in a first part of said upper limb and making a second set of measurements of the parameter in a second (different) part of said upper limb as a function of time. In this case, the method may further comprise comparing a measurement taken at a pre-determined point in time in said first part of the upper limb with the measurement taken in said second part of the upper limb at said pre-determined point in time, a difference (and optionally the magnitude of the difference) being suggestive of the presence, absence or severity of said neurovascular disease. This is a particularly effective method. Additionally or alternatively, the method may further comprise step (iv) may comprise comparing a measurement taken at a pre-determined point in time in said first part of the upper limb with a measurement taken in said second part of the upper limb at the same pre-determined point in time for several pre-determined points in time, the variation in difference over time between the measurement taken in the first part of the upper limb and the measurement taken in the second part of the upper limb being suggestive of the presence, absence or severity of said neurovascular disease.

The first and second parts of the upper limb may be distal and proximal regions of the same digit respectively, or may be a region of a digit and a region of the dorsum or palm respectively. For example, if the temperature of the distal region minus the temperature of the proximal region is less than 0°C a fixed period after vibration (say 10 minutes), then this may be indicative of the presence of neurovascular disease.

The step of comparing measurements may comprise calculating the difference between measurements.

The method may further comprise the step of making a first measurement of said parameter in said first part of the upper limb and making a second measurement of said parameter in said second part of the upper limb before step (ii), a difference in said first and second measurements being a pre-stimulation difference. The pre-stimulation difference may be compared to the differences in the measurements taken from the first and second parts of the upper limb during or after vibration of the upper limb. In this case, the period of time taken for the difference in the measurements taken from the first and second parts of the upper limb after vibration of the upper limb to return to the pre-stimulation difference may be suggestive of the absence, presence or severity of neurovascular disease. If the period of time is long, then this is suggestive of the presence of neurovascular disease. "Long" may be defined as a period of time to return to the pre-stimulation difference that is two standard deviations longer than the mean time.

Step (iv) preferably takes place subsequent to step (iii). It is more preferred that step (iv) commences immediately subsequent to step (iii).

The method may further comprise sensing a grasping force that the subject exerts on said handle during vibration. This allows grip strength to be sensed and measured.

The method may further comprise indicating the grasping force to the subject. This allows the subject to observe the grasping force. Indicating the grasping force to the subject may comprise indicating the magnitude of the grasping force relative to a predetermined level. This allows the subject to observe whether the grasping force is too low or too high.

The method may comprise the step of comparing the grasping force to a pre-determined value. For example, the grasping force may be compared to said pre-determined value over a period of time. In the event that the grasping force is lower than the pre-determined value for a period longer than a given pre-determined length of time, then the handle ceases to vibrate. This allows the method to be stopped in the event that the subject cannot maintain a sufficiently strong grip (which in itself may be suggestive of the presence of a neurovascular disease or disorder of a hand or arm).

The frequency of vibration may be approximately constant throughout step (iii).

The frequency of vibration may be from about 5Hz to about 1000Hz, preferably from about 5Hz to 500Hz and more preferably about 20Hz and/or about 200Hz.

The amplitude of vibration may be from about 2mm to 20mm and preferably from about 5mm to 10mm.

The amplitude of vibration may remain substantially constant throughout step (iii). Alternatively, the amplitude of vibration may be varied throughout step (iii).

If step (iii) is performed using a first frequency of vibration, then the method may further comprise the step of repeating steps (ii) to (iv) after a pre-determined period of time, wherein a second frequency of vibration is used during repeated step (iii).

The second frequency is preferably different from the first frequency, and it is further preferred that the second frequency is lower than the first frequency. Less serious HAVS may, for example, not be stimulated or show-up in response to a test at low frequencies, but more serious HAVS will respond in a positive manner to the test at both low and high frequencies. It is therefore beneficial to test at more than one frequency.

The method may further comprise the step of performing a cold provocation test a pre-determined period of time after step (iv) (or, in the case above where steps (ii) to (iv) are repeated, then the cold provocation test may be performed after repeated step (iv)).

The method of the present invention may use the apparatus in accordance with the present invention. Furthermore, the device provided in step (i) of the method of the present invention may have the features of the device for inducing vibrations in accordance with the present invention.

The present invention will be described by way of example only with reference to the following figures of which:
Figure 1 is a schematic diagram of one embodiment of an apparatus in accordance with the present invention;
Figure 2 is a schematic side elevation cross-sectional view through a device for inducing vibration used in the embodiment of Figure 1;
Figure 3 is a schematic end elevation cross-sectional view through the device of Figure 2;
Figure 4 is a schematic side elevation cross-sectional view of a further alternative embodiment of a device for inducing vibration;
Figure 5 is a schematic side elevation cross-sectional view of a further alternative embodiment of a device for inducing vibration;
Figure 6 shows the difference in temperature (as defined by the temperature of the distal region of the digit minus the temperature of the proximal region of the digit) in (Figure 6A) the little digit , (Figure 6B) the ring digit, (Figure 6C) the middle digit and (Figure 6D) the index digit as a function of time during a test using the apparatus shown in Figures 1, 2 and 3 of a subject suffering from HAVS and a control;
Figure 7 shows the mean temperatures of the digits and dorsum of the hand as a function of time during a test using the apparatus shown in Figures 1, 2 and 3 for a HAVS sufferer and a control subject;
Figure 8 shows the difference in temperature (as defined by the mean temperature of the digits minus the temperature of the dorsum) between the dorsum and digits as a function of time for a HAVS sufferer and a control subject; and
Figure 9 shows a schematic representation of a further embodiment of a device for inducing vibration.

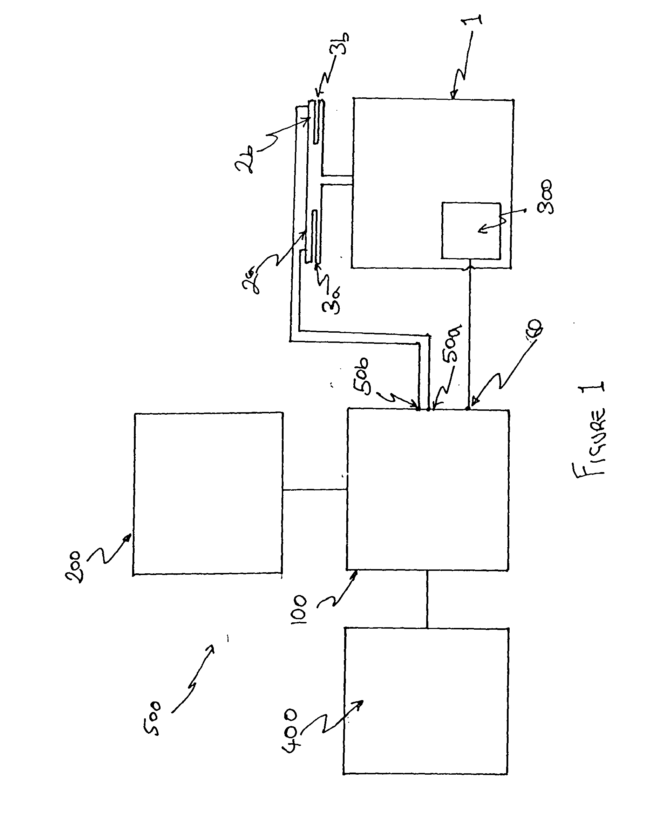

Figure 1 shows a schematic representation of an embodiment of an apparatus in accordance with the present invention. The apparatus 500 comprises a device 1 for inducing vibration in one or both of a hand and a forearm of a subject, a controller 100, an indicator 200 for indicating, inter alia, the strength of grip of a subject, and a temperature sensor 400 for sensing the temperature of the hand of a subject. The said device 1 comprises handles 2a, 2b, for a subject to grasp, each handle 2a, 2b being provided with a dynamometer 3a, 3b for measuring the grip strength in each hand of a subject. The said device 1 further comprises a motor 300 which creates vibration in the handles 2a, 2b as described in more detail below. The controller 100 (in this case a personal computer) is provided with inputs 50a, 50b (in this case, AD converters) to receive output signals or voltages from the respective dynamometers 3a, 3b, and an output 60 (in this case, a DA converter) to motor 300 that allows the controller 100 to control the operation of the motor 300. The motor 300 drives an axle (not shown) that causes vibrations of handles 2a, 2b. Controller 100 is in communication with the indicator 200 (in this case a conventional visual display unit). The operation of the apparatus will now be described in general terms with reference to Figure 1. Controller 100 provides electrical signals via output 60 to motor 300, thus controlling operation of the motor 300. Motor 300 drives the axle which, in turn, and subject to the handles being grasped with sufficient force, causes handles 2a, 2b to vibrate. A subject holds handles 2a, 2b during vibration. The strength of the grip in each hand is measured by dynamometers 3a, 3b, the grip strength in each hand determining the output signal or voltage of the respective dynamometer 3a, 3b which is transmitted to the controller 100 via inputs 50a, 50b. The grip strength of each hand is then displayed on the indicator 200. If a sufficiently strong grip is maintained by the subject, then the vibration is terminated after a pre-determined amount of time (such as 5 minutes). If a sufficiently strong grip is not maintained, then the vibration is terminated before the pre-determined amount of time is complete. After the cessation of vibration, the temperature of the hands of the subject is recorded as a function of time using the temperature sensor 400 (in this case, an infra-red camera). The temperature measurements are then used to determine whether the subject suffers from a neurovascular disease or disorder affecting one or both of a hand or arm.

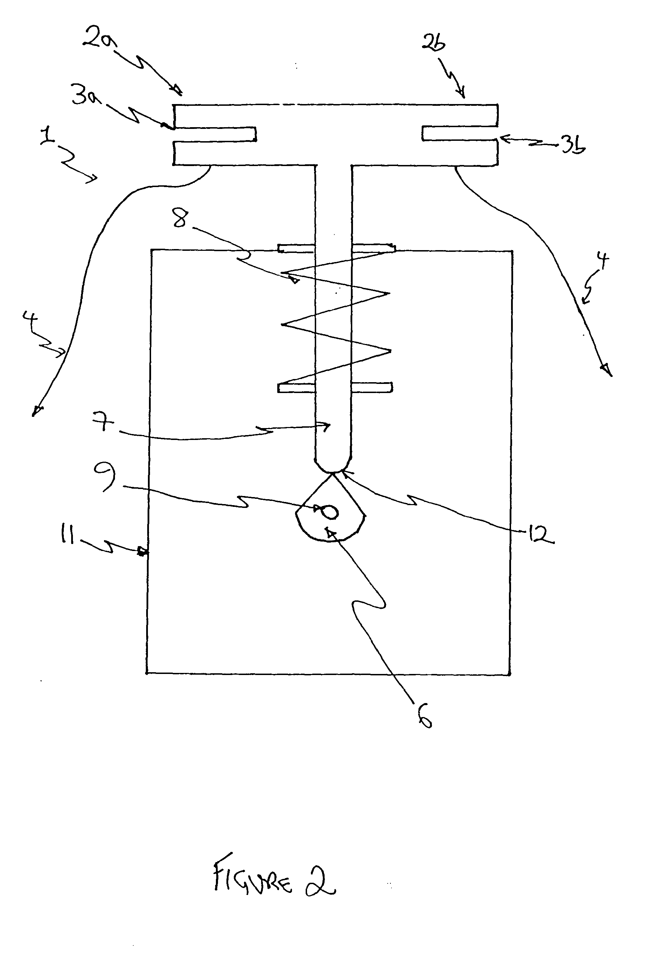

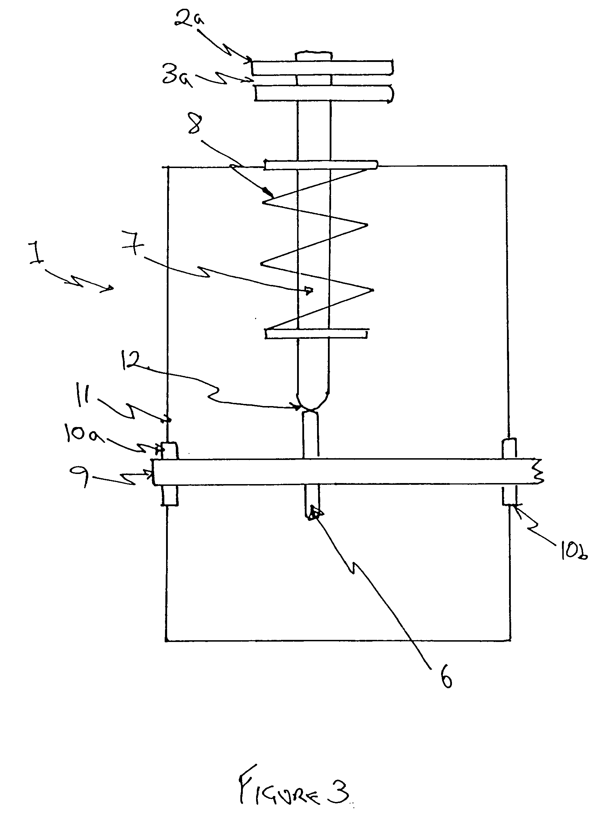

A more detailed description of the operation of the said device 1 is now described with reference to Figures 2 and 3. Figures 2 and 3 show the device 1 for inducing vibration as used in the apparatus of Figure 1. The device 1 comprises handles 2a, 2b for the subject to grasp and a mechanism for vibrating the handle 2. As mentioned previously, each handle 2a, 2b is provided with a dynamometer 3a, 3b that measures the strength of grip of the subject in each hand. The mechanism for vibrating the handle 2 comprises a cam 6 that is fixed to, and rotated by, an axle 9 that is turned by the motor (not visible), the operation of which is controlled by the controller (see Figure 1). A bearing race 10a, 10b is provided as an interface between the axle 9 and a housing 11 that is used to house the mechanism for vibrating the handle. The mechanism further comprises a cam follower 7 in the form of a steel rod. The end 12 of the cam follower 7 is urged into contact with the cam 6 for all orientations of the cam 6 by a helical compression spring 8. The cam follower 7 is connected to the handle 2 so that any motion of the cam follower 7 is transmitted to the handle 2. In use, the motor (not visible) causes the axle 9, and therefore the cam 6, to rotate. The end 12 of the cam follower 7 is urged into contact with the cam 6 for all orientations of the cam 6 and so rotation of the cam 6 causes translational (in this case, up and down) motion of the cam follower 7. The cam follower 7 is attached to the handle 2 and so the translational motion of the cam follower 7 is transmitted to the handle 2. The nature of the cam 6 ensures that the translational motion of the handle 2 is a repeated up and down motion. The rate of rotation of the axle 9 and cam 6 dictates the frequency of motion of the handle 2.

The operation of the apparatus and method of the present invention will now be described.

On arrival at the test station the maximum grip strength of the subject is measured; if it is below a predetermined limit, then the test is not performed, the indication of a weak grip strength indicating that the subject may be suffering from neurovascular disease of the upper limb. The controller 100 instructs the subject via the indicator 200 to grasp the handles 2a, 2b as hard as possible, the controller 100 recording the maximum output of each of the corresponding dynameters 3a, 3b, this maximum output corresponding to the maximum grip strength for each hand. Assuming that the subject can generate a sufficiently strong grip, then the hands and arms of the subject are exposed to the ambient environment for a given period (for example, five minutes) and a thermogram of the hands is taken using the temperature sensor 400. Two further thermograms are recorded at intervals of, for example, two to five minutes. If there is no cooling effect observed, then the subject may participate further in the test, otherwise the thermogram is repeated at two to five minute intervals until no further temperature change of the hands is observed. The handles 2a, 2b are then vibrated at a frequency of 200Hz for a period of one minute. During this period, the subject has to maintain an effective grip on the handle 2. The grip in each hand has to be maintained at or above a pre-determined level, in this case 60% of the maximum grip strength of the respective hand. If the grip strength falls below the pre-determined level for more than a given period, in this case 15 seconds, then the controller 100 causes the motor 300 to stop and thus the handles 2a, 2b to stop vibrating. Failure to maintain the indicated grip strength indicates that the respective hand and/or arm is weaker than expected and this in itself is indicative of some form of disorder. The test is terminated if the subject cannot maintain sufficient grip strength and no further temperature measurements of the hands are taken.

In the event that a sufficiently strong grip has been maintained throughout the one minute period of vibration, then, after vibration has ceased, thermograms are immediately taken of both hands. Further thermograms are recorded at different time intervals after cessation of vibration (for example, up to 10 minutes after cessation of vibration). A temperature difference as defined by the temperature of the distal region of the digit minus the temperature of the proximal region of the digit is calculated for each digit from each thermogram as a function of time.

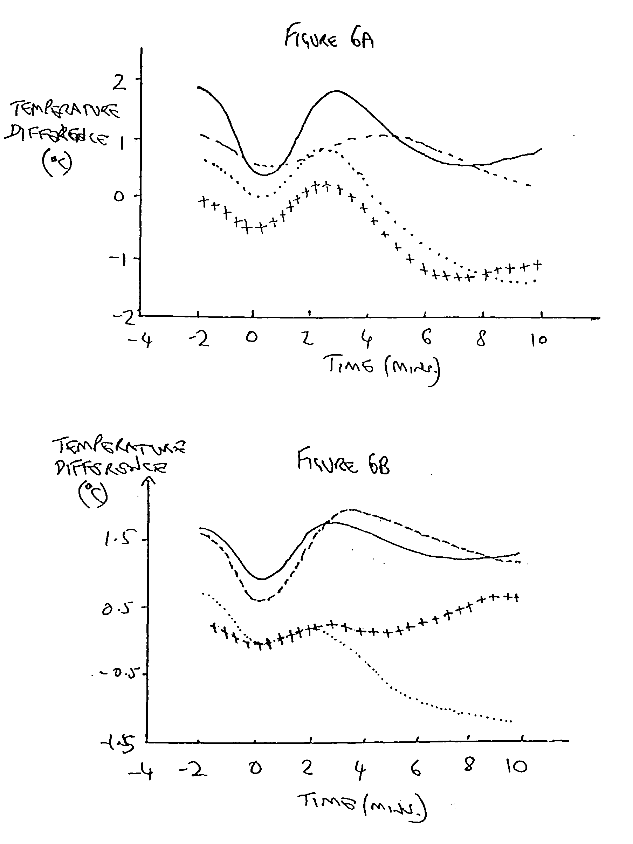

Figures 6A to 6D show variations in this temperature difference as function of time for various digits for a control subject and a sufferer of HAVS. The key for Figures 6A to 6D is as follows: "―" - difference for the respective digit of the left hand of a control subject; "- - -" difference for the respective digit of the right hand of the control subject; "++++" - difference for the respective digit of the left hand of a HAVS sufferer; "....." - difference for the respective digit of the right hand of a HAVS sufferer. The temperature of the hands is measured one minute before vibration (time=-2). The hands are subsequently vibrated for one minute (from time=-1 to 0), after which the temperature of the hands is measured periodically over a ten minute period (time=0 to 10). Referring to Figures 6a to 6d, RDIFF refers to the difference calculated using the noted digit of the right hand and LDIFF refers to the difference calculated using the noted digit of the left hand. In the case of the control, the value of the difference is positive ten minutes after cessation of vibration (time=10). In the case of the HAVS sufferer, the value of the difference is negative or very marginally positive for all digits apart from the left ring finger. The negative value of the difference is indicative of the presence of neurovascular disease.

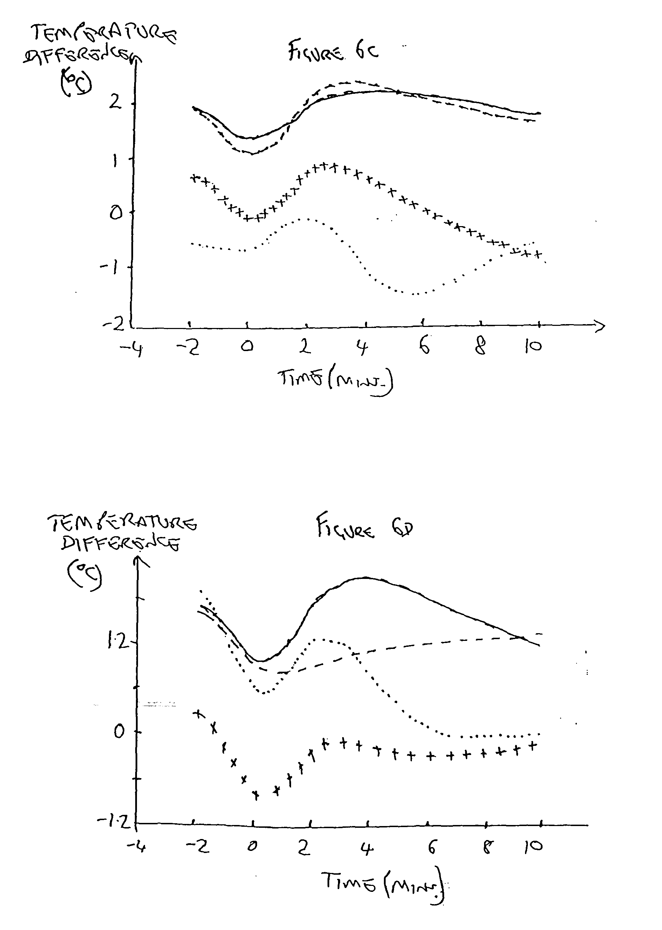

Alternatively, instead of measuring the difference in temperature between distal and proximal regions of a digit, it is possible to diagnose neurovascular disease by measuring the difference in temperature between the digits and the dorsum of the hand (the dorsum being the back of the main body of the hand), the difference being defined by the mean temperature of the digits minus the mean temperature of the dorsum. Figure 7 shows the mean temperatures of the digits and mean temperature of the dorsum for the right hand of a control subject and a HAVS sufferer as a function of time when subjected to the same testing regime described with respect to Figures 6A to 6D. The key to Figure 7 is as follows: "―" - mean temperature of the digits of a control subject; "- - - " - means temperature of the dorsum of the control subject; "......" - mean temperature of the digits of a HAVS sufferer; "+++" - means temperature of the dorsum of the HAVS sufferer. The results from the left hand have been omitted for clarity.

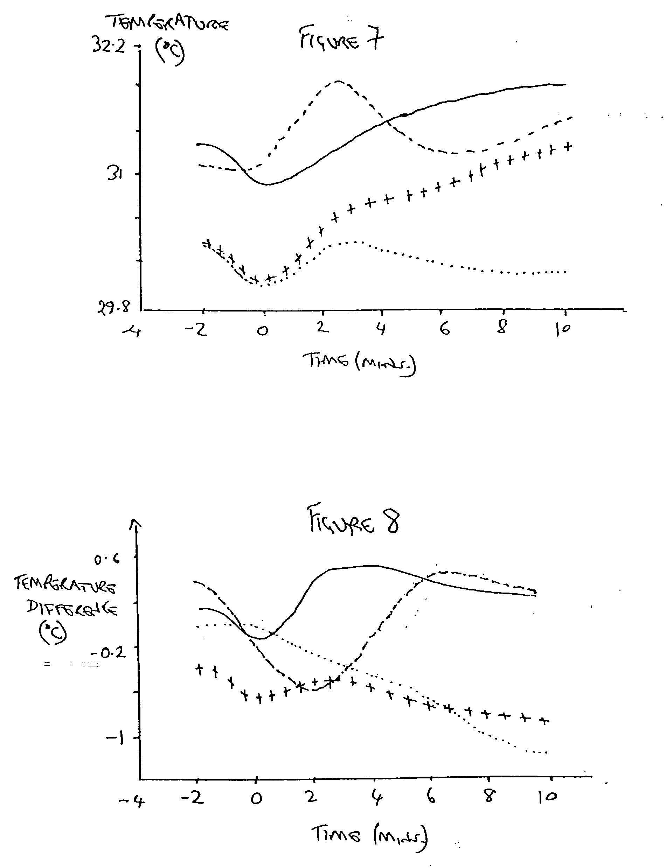

The measurements of Figure 7 may be used to calculate the variation over time of a temperature difference defined as the mean temperature of the digits minus the mean temperature of the dorsum for both the control and the HAVS sufferer. The variation of this temperature difference against time is shown in Figure 8 for both left and right hands. The key for Figure 8 is as follows: "―" - difference for the left hand of a control subject; "- --" difference for the right hand of the control subject; "++++" - difference for the left hand of a HAVS sufferer; "......" - difference for the right hand of the HAVS sufferer.. In the case of the HAVS sufferer, the difference is negative, and generally becomes more negative with increased time within the timeframe of the test. At time=10 minutes, the difference in the case of the control is positive.

The subject is at a later date (typically two weeks later) subjected to a corresponding test using a frequency of 20Hz, as opposed to 200Hz.

At a further later date (typically two weeks subsequent to the 20Hz test), the subject is subjected to a cold provocation test, which may be used as additional evidence concerning the disease or disorder status of a subject.

The temperature sensor 400 could be a camera or a point source. Ideally, the temperature sensor is thermally standardised or calibrated against a traceable standard.

Referring to Figures 2 and 3, the spring 8 may be omitted from the mechanism for vibrating the handle. This will produce a different type of oscillatory motion. Furthermore, the cam 6 may be of any suitable shape and may, for example, be shaped to reproduce the vibratory motion of a power tool that is suspected of inducing neurovascular disorders in the user. Alternatively, the shape of the cam 6 may be altered to produce a simple harmonic motion of the handle 2. The size of the cam 6 may be selected so as to alter the amplitude of the vibrations. The speed of rotation of the axle 9 may be selected to dictate the frequency of vibration of the handle 2.

The device 1 of Figures 2 and 3 is shown in a substantially vertical orientation and so produce essentially up-and-down vibrations. The device may simply be oriented on its side so as to produce back-and-forth vibrations; such a stimulus may replicate the use of power tools used in substantially horizontal configurations (such as the use of certain drills in mines).

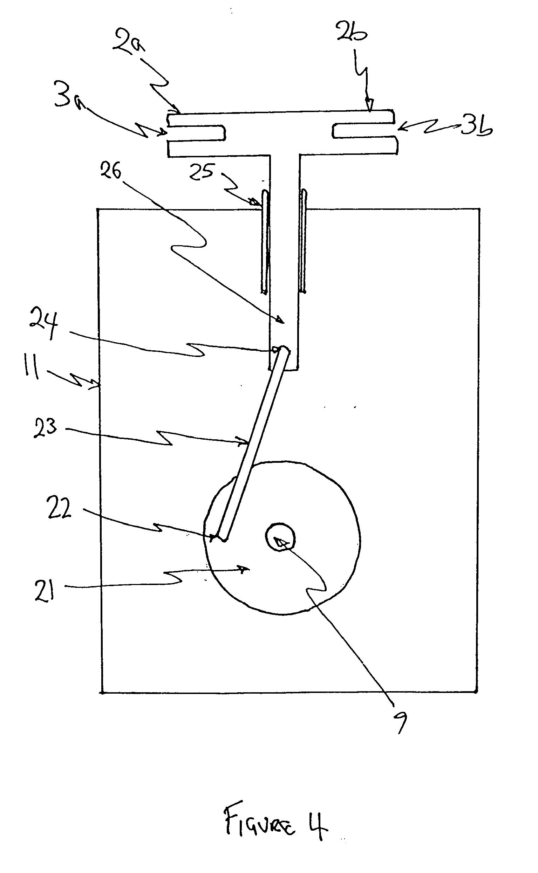

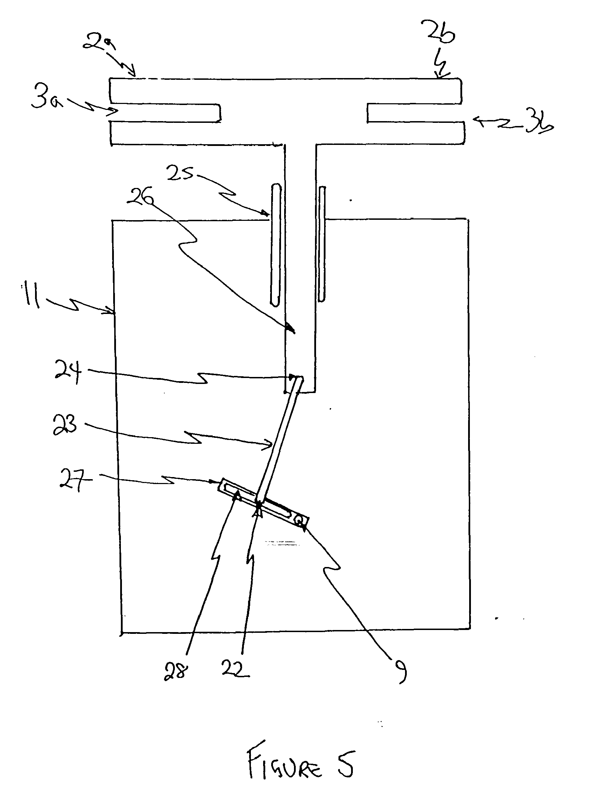

The cam/cam follower arrangement may be replaced by any suitable arrangement for producing a vibratory motion of the handle 2. For instance, the axle 9 may drive a wheel 21 to which is pivotally attached at an eccentric point a first end 22 of a rod 23 (see Figure 4). The second end 24 of the rod 23 is connected to a linking member 26 that is connected to the handles 2a, 2b. The linking member 26 is arranged within a guide 25 so that rotational motion of the axle 9 and thus wheel 21 causes the second end 24 of the rod 23 to undergo a motion that drives a reciprocating translational motion of the linking member 26 and thus a vibrational motion of the handles 2a, 2b. Alternatively, the wheel may be replaced by a radially extending crank arm 27 (see Figure 5). The first end 22 of the rod 23 may be movably connectable to the crank arm 27 such that the radial distance between the axle 9 and the point of attachment of the rod 23 to the crank arm 27 may be varied. The first end 22 of the rod 23 is associated with a longitudinally extending slot 28 provided in the crank arm 27. The first end 22 may be moved slidably along the slot 28, and then locked in position relative to the slot 28 using any convenient locking means (in this case, a nut and bolt). This allows the amplitude of vibration of the handles 2a, 2b to be varied between tests.

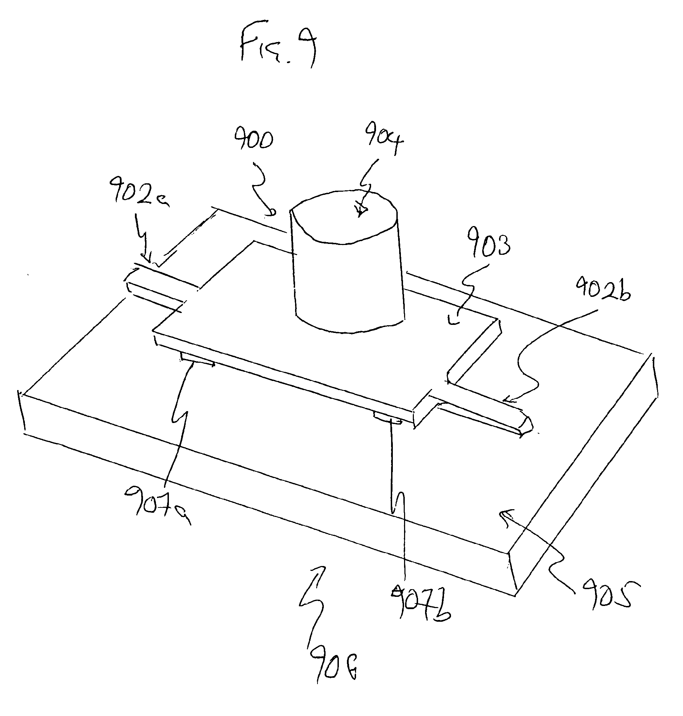

A further alternative device for inducing vibration is shown in Figure 9. The device is shown generally as reference numeral 900 and comprises two handles 902a, 902b. The handles are attached to a platform 903. As shown, the handles 902a, 902b are essentially parallel to one another. The platform is provided with a handle attachment point (not shown) to which one of the handles may be attached so that the handles are essentially mutually perpendicular. A ButtKicker LFE low frequency shaker 904 (available from The Guitammer Company Inc., Ohio 43086, USA) sits on, and is attached to, the platform 903. The platform 903 rests on four helical compression springs (two of which are shown as 907a, 907b) on top of a heavy base 905. The base 905 is made from cast iron and is provided with a slip-resistant rubber mat underneath. The base 905 is anchored to table 906.

An amplified low frequency signal (provided by an amplifier [not shown]) is supplied to the shaker 904. This causes the shaker to vibrate, thus causing vibration of the platform 903 and handles 902a, 902b. The springs ensure that the platform and handles can be shaken without shaking the base and table underneath. The heavy weight of the base assists in damping.

The shaker 904 comprises a reciprocating piston (not shown) that causes the shaking. It has been found that the device 900 has a greater longevity than some devices comprising a cam, possibly because of wear of the cam. Each of the handles 902a, 902b is also provided with a force sensor (not shown) for monitoring the strength of grip of a subject.

Those skilled in the art will realise that sensors other than thermography sensors may be used. For example, Doppler techniques could be used to measure bloodflow. Alternatively, the colour of the hand may be measured, the colour being indicative of blood flow. The temperature of the skin may be measured directly using non-contact thermometers.

## Claims

1. An apparatus for the diagnosis of neurovascular disease of at least part of an upper limb of a subject, the apparatus comprising:
(i) a device for inducing vibration in at least part of an upper limb of a subject, said device comprising a handle for the subject to grasp, wherein the handle is for vibrating at least part of an upper limb of the subject and
(ii) a sensor for measuring a parameter from which a measure of the bloodflow in at least part of the upper limb may be derived.

2. An apparatus according to claim 1 wherein the or each handle is provided with a force sensor for sensing the strength of the grip of the subject

3. An apparatus according to claim 2 further comprising at least one indicator for indicating the strength of the grip of the subject, the at least one indicator being in communication with the force sensor.

4. An apparatus according to claim 3 wherein, in use, the indicator indicates when the strength of the grip of the subject has reached a pre-determined value.

5. An apparatus according to any one of claims 2 to 4 further comprising a controller in communication with the device for inducing vibration, the force sensor, the bloodflow sensor and the indicator.

6. An apparatus according to any one of claims 2 to 5 wherein the apparatus is arranged, in use, to monitor whether the strength of grip of the subject is below a pre-determined limit for longer than a pre-determined time.

7. An apparatus according to any preceding claim wherein the apparatus is arranged to measure the bloodflow parameter during or after the vibration of the limb.

8. An apparatus according to any preceding claim wherein the frequency of vibration is in a range from about 5Hz to about 1000Hz.

9. An apparatus according to any preceding claim wherein the or each handle is associated with a mechanism for vibrating the handle, the mechanism comprising a cam and cam follower arrangement, wherein the cam follower is associated with a bias means that urges the cam follower into contact with the cam.

10. An apparatus according to any preceding claim wherein the mechanism for vibrating the or each handle comprises a shaker, wherein the or each handle is connected to or integral with a platform which is in contact with the shaker, said platform being associated with one or more elastically-deformable members which, in use, permit the platform to vibrate.

11. A device for inducing vibration in at least part of an upper limb of a subject suitable for use in an apparatus in accordance with claim 2 and either of claims 9 and 10.

12. A method of measuring a parameter from which a measure of bloodflow of a subject may be derived, the subject having an upper limb with a hand, the method comprising:
(i) providing a device with a vibratable handle;
(ii) the subject grasping the handle;
(iii) causing the grasped handle to be vibrated; and
(iv) during or subsequent to vibration of the hand, measuring a parameter in part of the upper limb from which a measure of the bloodflow may be derived.

13. A method according to claim 12 wherein step (iv) comprises making a first set of measurements of the parameter in a first part of the upper limb and making a second set of measurements of the parameter in a second, different, part of the upper limb as a function of time.

14. A method according to claim 13 wherein the first and second parts of the upper limb are distal and proximal regions of the same digit respectively, or a region of a digit and a region of the dorsum or palm respectively.

15. A method according to claim 13 or 14 further comprising the step of making a first measurement of said parameter in the first part of the upper limb and making a second measurement of said parameter in the second part of the upper limb before step (ii), the difference in the first and second measurements being a pre-stimulation difference.

16. A method according to any one of claims 12 to 15, the method further comprising sensing the grasping force that the subject exerts on said handle during vibration, and the step of comparing the grasping force to a pre-determined value.

17. A method according to any one of claims 12 to 16 wherein step (iii) is performed using a first frequency of vibration, the method further comprising the step of repeating steps (ii) to (iv) after a pre-determined period of time, wherein step (iii) is performed at a second frequency.
